# EUROPEAN PATENT APPLICATION

(11) **EP 4 332 215 A1**
(43) Date of publication of application: **06.03.2024**
(21) Application number: 22795832.9
(22) Date of filing: 27.04.2022
(51) Int. Cl.: C12N 5/071, C12N 5/0735, C12N 5/10

(54) **METHOD FOR PRODUCING CELLS**

(30) Priority: 28.04.2021 JP 2021075780
(71) Applicant: National University Corporation Tokyo Medical and Dental University, Tokyo 113-8510 (JP); Takeda Pharmaceutical Company Limited, Osaka-shi, Osaka 541-0045 (JP)
(72) Inventor: TAKEBE Takanori, Tokyo 113-8510 (JP); SAIKI Norikazu, Tokyo 113-8510 (JP)
(74) Representative: Jones, Nicholas Andrew
(86) International application number: PCT/JP2022/019017
(87) International publication number: WO 2022/230919

(57) **Abstract**

An object of the present invention is to provide an ever-better method for producing sinusoidal endothelial cells. The present invention provides a method for producing sinusoidal endothelial cells from sinusoidal endothelial progenitor cells, the method comprising the step of culturing the sinusoidal endothelial progenitor cells in a medium containing one or more substances selected from the interleukin 6 (IL-6) family, for example, oncostatin M (OSM), interleukin 6 (IL-6), or interleukin 11 (IL-11).

## Description

### Technical Field

The present invention relates to a method for producing sinusoidal endothelial cells from sinusoidal endothelial progenitor cells and a cell population comprising sinusoidal endothelial cells.

### Background Art

Capillaries, called "sinusoids", which intracellularly and intercellularly have pores of various sizes and have a basal membrane made of mere discontinuous layers, run throughout the liver or the bone marrow. Sinusoidal endothelial cells constituting the sinusoids are important for maintaining the functions of liver or bone marrow parenchymal cells. For example, with aim of achieving medicament development or regenerative medicine for diseases of various organs such as the liver, research and development are underway for preparing cell structures that reproduce three-dimensional structures of organs or complicated structures of blood vessels or other vascular channels by self-assembling cultured cells, i.e., organoids. If a structure corresponding to liver sinusoidal blood vessel can be reproduced in a liver organoid, it is expected that the value of using the organoid is further enhanced in such a way that the organoid can also be used in transplant operation or plasma protein (e.g., Factor VIII) production.

Sinusoidal endothelial cells of the liver or the like, when collected and separated from a living body, have the difficulty in maintaining their states or characteristics as sinusoidal endothelial cells due to poor states of the cells. Hence, the sinusoidal endothelial cells are generally produced by the induction of differentiation of their progenitor cells, i.e., sinusoidal endothelial progenitor cells, in a culture environment. For examples, the followings are known as methods for producing the sinusoidal endothelial cells. Non Patent Literature 1 states that human iPS cells were induced to differentiate into liver sinusoidal endothelial cell progenitors (LSEC progenitors), and then, the liver sinusoidal endothelial cell progenitors were treated for a long period (14 days) using a TGFb1 receptor inhibitor (A83-01) at a low oxygen concentration and thereby induced to differentiate into CD32± liver sinusoidal endothelial cells (LSECs). The purity of the liver sinusoidal endothelial cells in the cell population obtained by the method described in this Non Patent Literature 1 is on the order of less than 40%. Non Patent Literature 2 states that human ES cells were induced to differentiate into angioblasts, which were then treated using AMP, a TGFb inhibitor, bFGF, and the like at a low oxygen concentration or a normal oxygen concentration and thereby induced to differentiate into CD32+ liver sinusoidal endothelial cells. The purity of the liver sinusoidal endothelial cells in the cell population obtained by the method described in this Non Patent Literature 2 is 38.7% at the normal oxygen concentration and 90.1% at the low oxygen concentration.

### Citation List

### Non Patent Literature

Non Patent Literature 1: Koui et al., Stem Cell Reports, 9, 490-498, 2017
Non Patent Literature 2: Gage et al., Cell Stem Cell, 27, 254-269, 2020

### Summary of Invention

### Technical Problem

Methods for producing sinusoidal endothelial cells from sinusoidal endothelial progenitor cells are susceptible to improvement, for example, further improvement in purity of the sinusoidal endothelial cells in the resulting cell population.

An object of the present invention is to provide an ever-better method for producing sinusoidal endothelial cells.

### Solution to Problem

The present inventors have found that the culture of sinusoidal endothelial progenitor cells in a medium containing at least one substance (cytokine, etc.) selected from the interleukin 6 (IL-6) family, such as interleukin 6 (IL-6), oncostatin M (OSM), or interleukin 11 (IL-11), improves the purity of sinusoidal endothelial cells in the resulting cell population and can achieve a high purity (e.g., 95% or more) even if the culture is performed, particularly, at a normal oxygen concentration.

Specifically, the present invention includes at least the following items.
[1] A method for producing sinusoidal endothelial cells from sinusoidal endothelial progenitor cells, the method comprising the step of culturing the sinusoidal endothelial progenitor cells in a medium containing one or more substances selected from the interleukin 6 (IL-6) family.
[2] The method according to [1], wherein the one or more substances selected from the IL-6 family comprise at least interleukin 6 (IL-6), oncostatin M (OSM), interleukin 11 (IL-11), ciliary neurotrophic factor (CNTF), leukemia inhibitory factor (LIF), cardiotrophin 1 (CT-1), cardiotrophin-like cytokine 1 (CLC), interleukin 27 (IL-27), interleukin 35 (IL-35), interleukin 39 (IL-39), or a combination thereof.
[3] The method according to [1], wherein the one or more substances selected from the IL-6 family comprise at least IL-6, OSM, IL-11, or a combination thereof.
[3a] The method according to [1], wherein the one or more substances selected from the IL-6 family are IL-6, OSM, IL-11, or a combination thereof.
[4] The method according to [1], wherein the one or more substances selected from the IL-6 family comprise at least OSM.
[4a] The method according to [1], wherein the one or more substances selected from the IL-6 family are OSM.
[5] The method according to any one of [1] to [4], wherein the sinusoidal endothelial progenitor cells are liver sinusoidal endothelial progenitor cells, and the sinusoidal endothelial cells are liver sinusoidal endothelial cells.
[5a] The method according to any one of [1] to [4], wherein the liver sinusoidal endothelial progenitor cells are vitelline venous hemogenic endothelial cells or endocardial endothelial cells, and the sinusoidal endothelial cells are liver sinusoidal endothelial cells.
[6] The method according to [5], wherein the liver sinusoidal endothelial progenitor cells are vitelline venous hemogenic endothelial cells.
[7] The method according to any one of [1] to [6], wherein the medium further contains vascular endothelial growth factor (VEGF).
[8] The method according to any one of [1] to [7], wherein the sinusoidal endothelial progenitor cells are induced from pluripotent stem cells.
[9] A cell population comprising sinusoidal endothelial cells obtained by the method according to any one of [1] to [8].

### Advantageous Effects of Invention

The production method of the present invention can produce sinusoidal endothelial cells more efficiently than ever with a high purity, particularly, even at a normal oxygen concentration. Furthermore, the production method of the present invention can produce sufficiently matured sinusoidal endothelial cells expressing predetermined cell markers.

### Brief Description of Drawings

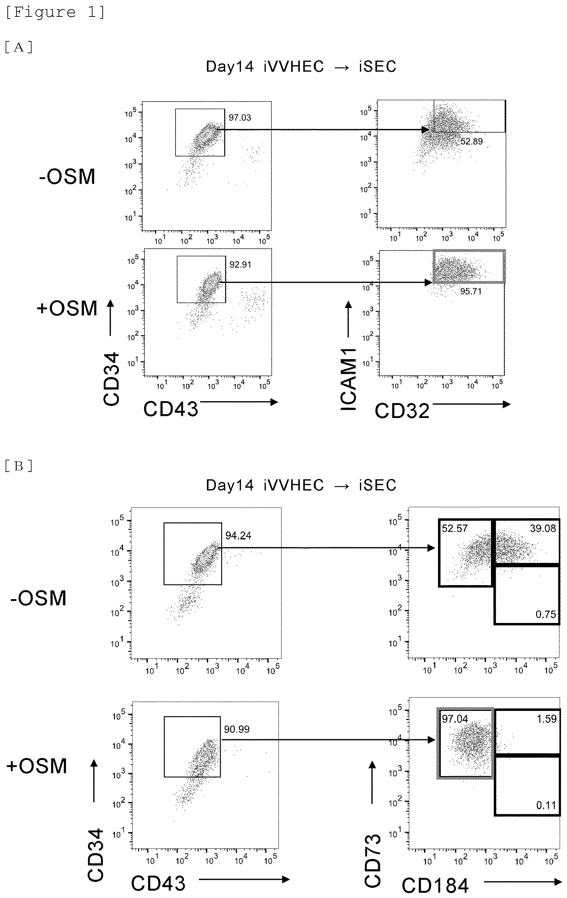

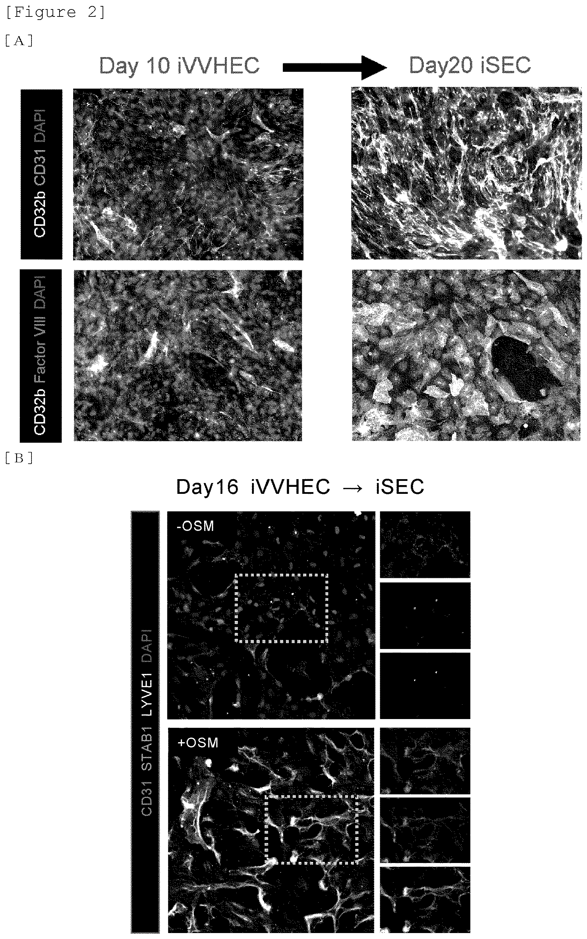

[Figure 1] Figure 1 shows results of [4] "Differentiation marker analysis of vascular endothelial cells using flow cytometry" in Example 1. "+OSM" depicts the case of adding oncostatin M as an IL-6 family substance to a medium, and "-OSM" depicts the case of adding no oncostatin M as a control (the same holds true for Figure 2). [A] The left boxes show results about the expression of CD34 (ordinate) and CD43 (abscissa) in vitelline venous hemogenic endothelial cells (hereinafter, referred to as "iWHECs") induced by the differentiation of iPS cells. The right boxes show results about the expression of ICAM1 (ordinate) and CD32 (abscissa) in (liver) sinusoidal endothelial cells (hereinafter, referred to as "iSECs") induced by the differentiation of iPS cells. [B] The left boxes show results about the expression of CD34 (ordinate) and CD43 (abscissa) in iVVHECs. The right boxes show results about the expression of CD73 (ordinate) and CD184 (abscissa) in iSECs.
[Figure 2] Figure 2 shows results of [5] "Differentiation marker analysis of cell population by immunocytostaining" in Example 1. [A] Upper boxes: Fluorescent images of CD32b, CD31, and DAPI. In color images, green which indicates the expression of CD31 and white which indicates the expression of CD32b are also observed to a given extent in cells in "Day 10 iVVHEC", whereas cells in "Day 20 iSEC" are strongly stained these two colors. Lower boxes: Fluorescent images of CD32b, Factor VIII, and DAPI. In color images, red which indicates the expression of Factor VIII and white which indicates the expression of CD32b are also observed to a given extent in cells in "Day 10 iWHEC", whereas cells in "Day 20 iSEC" are strongly stained these two colors. [B] Fluorescent images of CD31, STAB1, LYVE1, and DAPI. In color images, cells in iOMDay10 iWHECs" as cells in "+OSM" are also strongly stained red which indicates the expression of CD31, green which indicates the expression of STAB1, and white which indicates the expression of LYVE1, whereas red which indicates the expression of CD31 is observed to a given extent in cells in "-OSM", and however, green which indicates the expression of STAB1 and white which indicates the expression of LYVE1 are rarely observed therein.

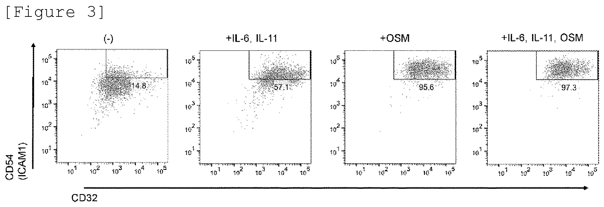

[Figure 3] Figure 3 shows results of "Differentiation marker analysis of vascular endothelial cells using flow cytometry" in Example 2. "(-)" depicts the case of adding no IL-6 family substance as a control; "+IL-6, IL-11" depicts the case of adding IL-6 and IL-11 as IL-6 family substances to a medium; "+OSM" depicts the case of adding oncostatin M as an IL-6 family substance to a medium; and "+IL-6, IL-11, OSM" depicts the case of adding IL-6, IL-11, and oncostatin M as IL-6 family substances to a medium.

### Description of Embodiments

### - Definition -

### Abbreviation

In the present specification, the following abbreviations are also used.
Liver sinusoidal endothelial cells: LSECs
Liver sinusoidal endothelial progenitor cells: LSEPCs
Vitelline venous hemogenic endothelial cells: VVHECs
Yolk sac mesoderm cells: YSMCs
Sinusoidal endothelial cells: SECs
Sinusoidal endothelial progenitor cells: SEPCs

### Pluripotent stem cell

In the present specification, the "pluripotent stem cells" refer to stem cells that can differentiate into tissues and cells having various different shapes and functions and have the ability to differentiate into cells of any lineage of the three germ layers (endoderm, mesoderm, and ectoderm). Examples of the pluripotent stem cells that may be used in the present invention include, but are not particularly limited to, induced pluripotent stem cells (also referred to as iPS cells), embryonic stem cells (also referred to as ES cells), embryonic stem cells derived from cloned embryos obtained by nuclear transplantation, spermatogonial stem cells, and embryonic germ cells.

The "induced pluripotent stem cells" (iPS cells) refer to cells that are obtained by reprograming mammalian somatic cells or undifferentiated stem cells by introducing particular factors (nuclear reprogramming factors). At present, there are various "induced pluripotent stem cells" and iPSCs established by Yamanaka, et al. by introducing the 4 factors Oct3/4, Sox2, Klf4, and c-Myc into mouse fibroblasts (Takahashi K, Yamanaka S., Cell, (2006) 126: 663-676); iPSCs derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors by a virus-free method (Okita K et al. Nat. Methods 2011 May; 8 (5): 409-12; and Okita K et al. Stem Cells. 31 (3) 458-66) may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used. In addition, any of induced pluripotent stem cells known in the art described in all published non patent literatures (e.g., Shi Y., Ding S., et al., Cell Stem Cell, (2008) Vol. 3, Issue 5, 568-574; Kim JB., Scholer HR., et al., Nature, (2008) 454, 646-650; and Huangfu D., Melton, DA., et al., Nature Biotechnology, (2008) 26, No 7, 795-797) or patent literatures (e.g., Japanese Unexamined Patent Application Publication No. 2008-307007, Japanese Unexamined Patent Application Publication No. 2008-283972, US2008/2336610, US2009/047263, WO2007/069666, WO2008/118220, WO2008/124133, WO2008/151058, WO2009/006930, WO2009/006997, and WO2009/007852).

Various iPS cell lines established by NIH, Riken (the Institute of Physical and Chemical Research), Kyoto University, and the like may be used as the induced pluripotent stem cells (iPS cells). Examples of the human iPS cell lines include HiPS-RIKEN-1A line, HiPS-RIKEN-2A line, HiPS-RIKEN-12A line, and Nips-B2 line from Riken, and 201B7 line, 253G1 line, 253G4 line, 409B2 line, 454E2 line, 606A1 line, 610B1 line, 625A4 line, 648A1 line, 1201C1 line, 1205D1 line, 1210B2 line, 1231A3 line, 1383D2 line, and 1383D6 line from Kyoto University. Alternatively, for example, cell lines of clinical grade provided by Kyoto University, Cellular Dynamics International, and the like, and cell lines for research or clinical uses prepared using these cell lines may be used.

The "embryonic stem cells" (ES cells) that may be used include mouse ESCs such as various mouse ES cell lines established by inGenious Targeting Laboratory, Riken (the Institute of Physical and Chemical Research), and the like, and human ES cells such as various human ES cell lines established by NIH, Riken, Kyoto University, and Cellartis. For example, CHB-1 to CHB-12 lines, RUES1 line, RUES2 line, and HUES1 to HUES28 lines from NIH, H1 line and H9 line from WisCell Research, KhES-1 line, KhES-2 line, KhES-3 line, KhES-4 line, KhES-5 line, SSES1 line, SSES2 line, and SSES3 line from Riken can be used as the human ES cell lines. Alternatively, for example, cell lines of clinical grade and cell lines for research or clinical use prepared using these cell lines may be used.

### Cell marker

In the present specification, the "cell marker" refers to a gene that is specifically expressed (positive marker) or not specifically expressed (negative marker) in a predetermined cell type, specifically, a substance that is produced (positive marker) or not produced (negative marker) as mRNA through the transcription of the gene in the genome or as a protein through the translation of the mRNA. The cell marker is preferably capable of being labeled (stained) with a fluorescent material, and is a protein (cell surface marker) that is expressed on cell surface and facilitates the detection, concentration, isolation, or the like of cells expressing the cell marker.

The term "positive" to a marker gene means that the expression level of mRNA or a protein of the gene is detectable by an approach general or known to those skilled in the art, or is higher than a predetermined threshold (background level, etc.). The term "strongly positive" to a marker gene means that the expression level of mRNA or a protein of the gene is higher than a predetermined threshold for determining whether to be "strongly positive" which is set to higher than a usual threshold for determining whether to be "positive". The term "negative" to a marker gene means that the expression level of mRNA or a protein of the gene is not detectable by an approach general or known to those skilled in the art, or is lower than a predetermined threshold (background level, etc.).

Whether to be positive or negative to the cell marker can be determined from qualitative or quantitative results by an approach general or known to those skilled in the art. The cell marker as a protein can be detected or its expression level can be measured, by use of immunoassay using an antibody specific for the protein, for example, ELISA, immunostaining, flow cytometry, or the like. The cell marker as mRNA can be detected or its expression level can be measured, by use of assay using a nucleic acid specific for the mRNA, for example, RT-PCR (including quantitative PCR), a microarray, a biochip, or the like.

The cells used in the present invention may be derived from a human or may be derived from a nonhuman animal, for example, a mammal such as a mouse, a rat, a dog, a pig, or a monkey. When an organ organoid or a three-dimensional organ prepared using the cell population of the present invention is used for the purpose of, for example, transplantation in a human or the development of a medicament for humans, the cells are preferably derived from a human.

In the present specification, an item described in a singular form and the item described in a plural form as to a cell, a compound, or other substances can be interpreted interchangeably with each other unless otherwise specified.

The term "comprise", "include", "contain", etc. refers to inclusion of the element(s) following the word without limitations thereto. Thus, this suggests inclusion of the element(s) following the word, but does not suggest exclusion of any other element. On the other hand, the term "consisting of", etc. means inclusion of every element following the term and a limitation thereto. Thus, the term "consist(s) of" or "consisting of" indicates that the enumerated element(s) is required or essential, and substantially no other elements exist. The term "consist(s) essentially of" or "consisting essentially of" means inclusion of any element following the term and a limitation of other elements to those influencing neither the activity nor the effect defined in the present disclosure as to the element following the term. Thus, the term "consisting essentially of", etc. indicates that the enumerated element(s) is required or essential, but other elements are optional and may or may not exist depending on whether to affect the activity or the effect of the enumerated element(s).

### - Method for producing cells -

The method for producing cells according to the present invention is a method for producing sinusoidal endothelial cells (SECs) from sinusoidal endothelial progenitor cells (SEPCs), the method comprising the step of culturing the SEPCs in a medium containing one or more substances selected from the interleukin 6 (IL-6) family (hereinafter, also referred to as "IL-6 family substances").

The "sinusoidal endothelial cells" (SECs) refer to vascular endothelial cells constituting various "sinusoids" of the liver, the bone marrow, the pituitary gland, and the like. SECs have a stomal structure (fenestrated structure, average diameter: 107 ± 1.5 nm, 528 ± 142 pores per cm³; see https://www.nature.com/articles/gt200860) where material exchange is performed. In the present invention, not only sinusoidal endothelial cells of the liver (liver sinusoidal endothelial cells: LSECs) but sinusoidal endothelial cells (SECs) in various organs or tissues, other than the liver, such as the bone marrow, the pituitary gland, the spleen, the renal glomerulus, and the adrenal gland can be intended for the production method of the present invention.

The "sinusoidal endothelial progenitor cells" (SEPCs) refer to progenitor cells having the ability to differentiate into the sinusoidal endothelial cells (SECs) mentioned above. As in SECs, not only progenitor cells of sinusoidal endothelial cells of the liver (liver sinusoidal endothelial progenitor cells) but progenitor cells of sinusoidal endothelial cells in various organs or tissues, other than the liver, such as the bone marrow, the pituitary gland, the spleen, the renal glomerulus, and the adrenal gland can be used as SEPCs in the production method of the present invention. In other words, on the basis of a commonality among various SEPCs and a commonality among various SECs, the production method of the present invention can utilize SEPCs derived from the liver and other organs or tissues for inducing their differentiation into corresponding SECs derived from the liver and other organs or tissues.

In one aspect of the present invention, the sinusoidal endothelial progenitor cells (SEPCs) are liver sinusoidal endothelial progenitor cells (LSEPCs), and the sinusoidal endothelial cells (SECs) are liver sinusoidal endothelial cells (LSECs).

The liver sinusoidal endothelial cells (LSECs) are cells known to have CD32 (more specifically, CD32b) and ICAM1 (CD54) as major cell markers. The cells are known to additionally have, for example, Factor VIII, STAB1, and LYVE1 as cell markers. Specifically, in the case of being positive to CD32 and strongly positive to ICAM1 and, preferably, being further positive to at least one (more preferably two, particularly preferably three) of Factor VIII, STAB1, and LYVE1, the cells can be confirmed to be LSECs (when the conditions are not satisfied, the cells are cells other than LSECs). A positive marker CD31 for vascular endothelial cells is also a positive marker for LSECs and other SECs. Sinusoids typically have a venous capillary structure, and a positive marker CD73 and a negative marker CXCR4 for venous vascular endothelial cells are also a positive marker and a negative marker, respectively, for LSECs and other SECs.

The "liver sinusoidal endothelial progenitor cells" (LSEPCs) refer to cells that correspond to progenitor cells of LSECs and have the ability to differentiate into liver sinusoidal endothelial cells (LSECs). Examples of LSEPCs include vitelline venous hemogenic endothelial cells (VVHECs) and endocardial endothelial cells (EECs).

The vitelline venous hemogenic endothelial cells (VVHECs) are hemogenic endothelial cells (HECs) derived from yolk sac mesoderm and are known as cells responsible for hemogenesis in yolk sac in early fetal life. WHECs have CD34 as a major cell marker, and the cells are known to additionally have, for example, FN1, ACTA2, and LYVE1, and venous markers NR2F2, APLNR, and PROX1 as cell markers. Specifically, in the case of being positive to at least CD34 and, preferably, being further positive to at least one cell marker selected from the group consisting of FN1, ACTA2, LYVE1, NR2F2, APLNR, and PROX1, the cells can be confirmed to be WHECs (when the conditions are not satisfied, the cells are cells other than WHECs). A positive marker CD31 for vascular endothelial cells is also a positive marker for VVHECs. CD43 is also a negative marker for VVHECs.

The endocardial endothelial cells (EECs) are another endothelial cell that is present *in vivo* together with vascular endothelial cells. EECs have CD34, CD31, VE-cadherin, and the like as major cell markers, and the cells are known to additionally have NFATC1, NRG1, NKX2-5, and the like as cell markers. Specifically, in the case of being positive to at least one cell marker selected from the group consisting of CD34, CD31, and VE-cadherin and, preferably, further positive to at least one cell marker selected from the group consisting of NFATC1, NRG1, and NKX2-5, the cells can be confirmed to be EECs (when the conditions are not satisfied, the cells are cells other than EECs).

The sinusoidal endothelial progenitor cells (SEPCs) for use in the method for producing cells according to the present invention can be provided by an approach known in the art. For example, pluripotent stem cells such as iPS cells or ES cells can be induced to differentiate into LSEPCs or other SEPCs (through lateral plate mesodermal cells or the like) in accordance with an approach known in the art. Examples of such a method include a method of inducing the differentiation of pluripotent stem cells such as iPS cells into lateral plate mesodermal cells, and then further inducing their differentiation into yolk venous hemogenic endothelial cells (VVHECs) (see the section [2] Preparation of human hemogenic endothelial cells in Examples of WO2020/203713). Another example thereof includes a method of inducing the differentiation of yolk sac mesoderm cells (YSMCs) into vascular endothelial progenitor cells (EPCs) with reference to Ohta, R. et al., J. Vis. Exp. (148), e59823, doi:10.3791/59823 (2019), and then inducing the differentiation of the EPCs into WHECs with reference to Matsubara et al., Biochemical and Biophysical Research Communications, 515 (1), 2019.

In the method for producing cells according to the present invention, SECs can be obtained by culturing SEPCs in a medium containing an IL-6 family substance. The culture of SEPCs is usually performed by two-dimensional culture (plane culture). The culture container for two-dimensional culture is not particularly limited, and a dish, a flask, a microplate, a cell culture sheet such as trade name "OptiCell" (Nunc/Thermo Fisher Scientific Inc.), or the like can be used. The culture container is preferably surface-treated in order to improve adhesiveness to cells (hydrophilicity), or coated with a substrate for cell adhesion such as collagen, gelatin, poly-L-lysine, poly-D-lysine, laminin, fibronectin, Matrigel (e.g., BD Matrigel (Nippon Becton Dickinson Co., Ltd.)), or vitronectin.

The substance belonging to the "IL-6 family" refers to, for example, a cytokine sharing gp130 as a signal transducer (receptor). For example, interleukin 6 (IL-6), interleukin 11 (IL-11), interleukin 27 (IL-27), interleukin 35 (IL-35), interleukin 39 (IL-39), leukemia inhibitory factor (LIF), oncostatin M (OSM), cardiotrophin 1 (CT-1), ciliary neurotrophic factor (CNTF), and cardiotrophin-like cytokine 1 (cardiotorophin-like cytokine: CLC) have been known so far as cytokines and the like of the IL-6 family. In the present invention, any one member selected from the IL-6 family can be used singly as the IL-6 family substance, or a plurality thereof can be used in combination.

In one aspect of the present invention, the IL-6 family substance preferably comprises at least IL-6, OSM, IL-11, or an arbitrary combination selected therefrom, and more preferably comprises at least OSM. Examples of the arbitrary combination include a combination of IL-6 and IL-11, and a combination of IL-6, IL-11, and OSM.

The concentration of the IL-6 family substance in a medium is not particularly limited and can be appropriately adjusted according to the working effect of the present invention, particularly, the induction efficiency of differentiation of LSEPCs into LSECs, a desired purity of LSECs in the resulting cell population, and the composition of the medium (basal medium and other components used in combination with the IL-6 family substance), etc. In one aspect of the present invention, the concentration of the IL-6 family substance in a medium can be set to within the range of, for example, 5 to 100 µM, preferably 5 to 20 µM.

Examples of the basal medium for preparing the medium used in the present invention include DMEM/F-12 (Gibco), Stempro-34 SFM (Gibco), Essential 6 medium (Gibco), Essential 8 medium (Gibco), EGM (Lonza Group AG), EGM-2 (Lonza Group AG), EGM-2 MV (Lonza Group AG), HCM (Gibco), Bullet Kit (Lonza Group AG), VascuLife EnGS Comp Kit (LCT), Human Endothelial-SFM Basal Growth Medium (Invitrogen Corp.), and human microvascular endothelial cell proliferation medium (Toyobo Co., Ltd.).

Examples of an additive (other than the IL-6 family substance) for preparing the medium used in the present invention include one or more members selected from the group consisting of B27 Supplements (Gibco), BMP4 (bone morphogenetic protein 4), GSKβ inhibitors (e.g., CHIR99021), VEGF (vascular endothelial growth factor), FGF2 (fibroblast growth factor (also referred to as bFGF (basic fibroblast growth factor))), Folskolin, SCF (stem cell factor), TGFβ receptor inhibitors (e.g., SB431542), ROCK inhibitors (e.g., Y-27632), Flt-3L (Fms-related tyrosine kinase 3 ligand), IL-3 (interleukin 3), TPO (thrombopoietin), hEGF (recombinant human epithelial growth factor), hydrocortisone, ascorbic acid, IGF1, FBS (fetal bovine serum), antibiotics (e.g., gentamycin and amphotericin B), heparin, L-glutamine, Phenol Red, and BBE.

In one aspect of the present invention, the medium for use in the method for producing cells according to the present invention preferably contains vascular endothelial growth factor (VEGF). The concentration of VEGF in the medium can be set to within the range of, for example, 5 to 80 µM, preferably 5 to 10 µM.

The culture of SEPCs according to the present invention can be performed at a normal oxygen concentration (concentration that is not particularly adjusted, for example, approximately 210). In this case as well, a cell population having a higher purity of SECs than ever can be obtained. The culture may be performed at a low oxygen concentration (e.g., less than 10% or approximately 5% as an example).

The culture period of SEPCs according to the present invention is not particularly limited and can be, for example, a period in which the number of SECs in a cell population is sufficiently increased to reach a desired purity. The culture period is usually 6 to 20 days, preferably 6 to 14 days.

Other technical items regarding the culture of SEPCs can be appropriately adjusted by those skilled in the art and, if necessary, can abide by a method known in the art for producing SECs from SEPCs or can be a version thereof modified so as to adapt to the present invention.

### - Cell population -

The cell population of the present invention is a cell population comprising SECs obtained by the aforementioned method for producing cells according to the present invention. The method for producing cells in the present specification can produce a cell population with a relatively high purity of SECs (without the need of additional steps such as refinement, purification, and concentration).

The purity of SECs in the cell population is not particularly limited, and a desired degree of the purity can be achieved according to the purpose, etc. of the cell population. The purity can be, for example, 50% or higher, 60% or higher, 70% or higher, 80% or higher, 85% or higher, 90% or higher, or 95% or higher.

The "purity" of particular cells (in the present invention, SECs) is a numeric value represented by the ratio of the number of cells concerned to the total number of cells in the cell population and can be measured by a general approach using, for example, flow cytometry. In the present invention as well, for example, a sample can be collected from a cell population obtained by the method for producing cells according to the present invention and subjected to fluorescent immunostaining targeting cell markers for SECs (e.g., the markers separately described in the present specification), followed by measurement using flow cytometry to determine the purity of SECs in the cell population.

The purpose of the cell population of the present invention is not particularly limited, and the cell population can be used for various purposes using SECs. For example, the (SECs contained in) cell population of the present invention can be used for preparing an organoid or a three-dimensional organ of an organ or a tissue having sinusoids of the liver, the bone marrow, or the like.

### Examples

In the following Examples, "Day" refers to the number of days from the start of induction of differentiation of a human iPS cell colony. "Day 0" is the start of culture in the step [1] given below.

### [Example 1] Preparation of liver sinusoidal endothelial cells by addition of oncostatin M

### [Experimental method]

### [1] Preparation of human yolk sac mesoderm cells

Human iPS cells (625A4; Center for iPS Cell Research and Application (CiRA), Kyoto University) were cultured at 37°C under 5% CO₂ in AK02N (Ajinomoto Co., Inc.) (10 cm dish; 8 ml, 24-well plate; 0.5 ml) until iPS cell colonies of 500 to 700 µm in diameter were formed (for approximately 6 to 7 days). The obtained colonies were cultured at 37°C for 2 days under 5% CO₂ in Essential 8 medium (Gibco) (10 cm dish; 8 ml, 24-well plate; 0.5 ml) supplemented with BMP4 (80 ng/ml), VEGF (80 ng/ml), and CHIR99021 (2 µM) to prepare human yolk sac mesoderm cells (Days 0 to 2).

### [2] Preparation of human vitelline venous hemogenic endothelial cells

Following the [1] preparation of human yolk sac mesoderm cells, the medium was replaced with Essential 6 medium (Gibco) (10 cm dish; 8 ml) supplemented with VEGF (80 ng/ml), FGF2 (25 ng/ml), SCF (50 ng/ml), and SB431542 (2 µM), and hemangioblasts were induced by further culture at 37°C for 2 days under 5% CO₂ (Days 3 to 4). Then, the medium was replaced with Stempro-34 SFM (Gibco) (10 cm dish; 8 ml) supplemented with VEGF (80 ng/ml), SCF (50 ng/ml), Flt-3L (50 ng/ml), IL-3 (50 ng/ml), IL-6 (50 ng/ml), and TPO (5 ng/ml), and the cells were cultured at 37°C for 2 days under 5% CO₂ (Days 5 to 6). Then, the medium was replaced with a medium of the composition described above except for VEGF, and CD34-positive and CD32-positive vitelline venous hemogenic endothelial cells (iWHECs) were induced by culture at 37°C for 2 days under 5% CO₂ (Days 7 to 8).

### [3] Preparation of human liver sinusoidal endothelial cells

The cell population containing the CD34-positive and CD32-positive human vitelline venous hemogenic endothelial cells obtained by the step of [2] was reacted with TrypLe Express (Gibco) at 37°C for 15 minutes. Then, the cells were recovered into a 15 mL tube while dissociated by pipetting with P1000 micropipette. After addition of 7 mL of Stempro-34 SFM (Gibco) medium, the cells were centrifuged at 1000 rpm at room temperature for 5 minutes. After removal of the supernatant, the cells were suspended in a PBS(-) buffer containing 1 mM EDTA and 4% FBS, and cell counting was carried out. 1 × 10⁸ or less cells were dispensed, centrifuged under the same conditions as above, and then suspended in 300 µl of a PBS(-) buffer containing 1 mM EDTA and 4% FBS. 1 µl of CD34 MicroBead Kit (Miltenyi Biotec) was added per 1 × 10⁶ cells, mixed, and left standing on ice for 30 minutes. Subsequently, the cells were washed twice with a PBS(-) buffer containing 1 mM EDTA and 4% FBS and then suspended in 500 µl of the same buffer as above. CD34-positive vitelline venous hemogenic endothelial cells were refined and obtained using QuadroMACS Separator and LS Column (Miltenyi Biotec). The refined cell suspension was centrifuged at 1000 rpm at room temperature for 5 minutes. After removal of the supernatant, the cells were suspended in a liver sinusoidal endothelial cell induction medium obtained by mixing HCM (Lonza Group AG) medium supplemented with 5% FBS Gold (Biosera) and OSM (20 ng/ml) (R&D Systems, Inc.) with EGM (Lonza Group AG) medium at a volume ratio of 1:1, and adding thereto VEGF (5 ng/mL) and Y-27632 (10 µM) (FUJIFILM Wako Pure Chemical Corp.), then inoculated at 1 × 10⁵ cells and 2 × 10⁵ cells to a 24-well plate and a 12-well plate, respectively, coated at 37°C for 30 minutes with Matrigel (BD Pharmingen) diluted 50-fold with PBS(-), and cultured at 37°C for 6 days or longer under 5% CO₂ (Days 9 to 14 and later) to obtain a cell population containing liver sinusoidal endothelial cells. Medium replacement was performed using the liver sinusoidal endothelial cell induction medium except for Y-27632 (10 µM) on a day following inoculation, and then carried out every two days.

A cell population containing liver sinusoidal endothelial cells was obtained as a control by the culture of vitelline venous hemogenic endothelial cells in the same manner as above except that no OSM (final concentration: 10 ng/ml) was added.

### [4] Differentiation marker analysis of vascular endothelial cells using flow cytometry

After induction of differentiation using a 12-well plate in the steps of [1] to [3], the medium was removed, and the cells were washed twice with PBS(-) (Gibco). Then, TrypLE Express (Gibco) was added at a volume of 1 mL/well and left at 37°C for 15 minutes. Then, the cells were recovered into a 15 mL tube while dissociated by pipetting with P1000 micropipette. After addition of 5 mL of Stempro-34 SFM (Gibco) medium, the cells were centrifuged at 1000 rpm at room temperature for 5 minutes. After removal of the supernatant, the cells were washed once using a PBS(-) buffer containing 1 mM EDTA and 4% FBS. Then, the cells were suspended in 50 µl of a primary antibody solution (1/50 PE-CD32 (BioLegend, Inc., Cat: 303205), BV421-CD34 (BD Biosciences, Cat: 562577), APC-CD54 (BD Biosciences, Cat: 559771), and BV786-CD43(BD Biosciences, Cat: 744662) in BD Horizon Brilliant(TM) Stain Buffer) and left standing on ice for 30 minutes. Subsequently, the cells were washed twice with a PBS(-) buffer containing 1 mM EDTA and 4% FBS, then suspended in a PBS(-) buffer containing 1/1000 DAPI, 1 mM EDTA, and 4% FBS, and transferred to a tube with a 40 µm cell strainer cap. The differentiation markers were analyzed by BD LSRFortessa flow cytometry using the obtained antibody-stained cell suspension. Data obtained from flow cytometry was analyzed using FlowJo 10.7.1 (BD).

The results are shown in Figure 1[A]. As is evident, the production method of the present invention using oncostatin M produced a cell population containing CD32+ICAM1 (CD54)++ LSECs (iSECs) with a very high purity (95% or higher) from CD34+ VVHECs (iVVHECs).

An antibody-stained cell suspension was obtained in the same manner as above except that the primary antibody solution was changed to 1/50 PE-CD184 (BioLegend, Inc., Cat: 306506), BV421-CD34 (BD Biosciences, Cat: 562577), APC-CD73 (Miltenyi Biotec, Cat: 130-095-183), and BV786-CD43 (BD Biosciences, Cat: 744662) in BD Horizon Brilliant(TM) Stain Buffer. Differentiation markers were analyzed by flow cytometry. The results are shown in Figure 1[B]. The cell population obtained by the production method of the present invention using oncostatin M contained CD73+CXCR4- cells, demonstrating that oncostatin induced a surface marker profile of the venous vascular endothelial cells.

### [5] Differentiation marker analysis of cell population by immunocytostaining

After induction of differentiation using a 24-well plate in the steps of [1] to [3], the medium was removed, and the cells were washed once with PBS(-) (Gibco). Then, 4% paraformaldehyde was added at a volume of 500 µl/24 wells to the cells and left at room temperature for 15 minutes. Then, the cells were washed three times with PBS(-) and preserved overnight at 4°C. Then, PROTEIN BLOCK SERUM-FREE blocking solution [DAKO, X909] was added at a volume of 500 µl/24 wells to the cells and left at room temperature for 60 minutes for blocking. Then, different combinations on a well basis of primary antibodies against the target antigen (an antibody set of 1/100 Anti-Factor VIII (Abeam plc, ab41188) and 1/500 Anti-CD32b (Abeam plc, ab151497) or an antibody set of 1/50 Anti-CD31 (Abeam plc, ab24590) and 1/500 Anti-CD32b (Abeam plc, ab151497)) were added to PBS(-) containing 0.1% donkey serum, and this primary antibody solution was added at a volume of 500 µl/24 wells to each well and left standing at room temperature for 60 minutes or overnight at 4°C. Subsequently, after washing three times with PBS(-), a secondary antibody (1/1000, Alexa Flour 555, and 1/1000, Alexa Flour 647) and 1/1000 DAPI-HCB Hycult Biotech, HM2167) corresponding to each primary antibody was added to a 1% blocking solution in PBS(-), and this secondary antibody solution was added at a volume of 500 µl/24 wells and incubated at room temperature for 60 minutes. Then, after washing three times with PBS(-), PBS(-) was added at a volume of 500 µl/24 wells and held. Fluorescent images were obtained under BZ-X700 fluorescence microscope (Keyence Corp.).

The results are shown in Figure 2[A]. As is evident, LSECs (iSECs) contained in the cell population obtained by the production method of the present invention not only expressed CD31 and CD32 on cell surface (i.e., were positive to these markers), but secreted a large amount of Factor VIII protein.

Immunocytostaining was performed in the same manner as above except that the primary antibody solution was changed to an antibody set of 1/100 Anti- Stabilin-1 (Novus Biologicals, LLC, H00023166-M05), 1/100 Anti-CD31 (Abeam plc, ab28364), and 1/100 Anti-LYVE1 (R&D Systems, Inc., AF2089); and the secondary antibody solution was changed to a solution supplemented with a secondary antibody corresponding to each primary antibody (1/1000, Alexa Flour 555, 1/1000, Alexa Flour 594, 1/1000, Alexa Flour 647) and 1/1000 DAPI-HCB Hycult Biotech, HM2167). Cells that were not supplemented with oncostatin M in the steps of [1] to [3] were also subjected as a control to immunocytostaining. Fluorescent images were obtained under LSM880 confocal microscope (Zeiss). The results are shown in Figure 2[B]. As is evident, LSECs (iSECs) contained in the cell population obtained by the production method of the present invention also expressed STAB1 and LYVE1 proteins as sinusoidal endothelial markers, in addition to CD32 and Factor VIII protein mentioned above.

### [Example 2] Preparation of liver sinusoidal endothelial cells by addition of IL-6 and IL-11

The steps of [1] and [2] were performed in the same manner as in Example 1. Then, in the step of [3], any of 1) to 3) given below was used as the "liver sinusoidal endothelial cell induction medium" to obtain a cell population containing liver sinusoidal endothelial cells. 2) was repetition of substantially the same step as in Example 1.
1) A liver sinusoidal endothelial cell induction medium obtained by mixing HCM (Lonza Group AG) medium supplemented with 5% FBS Gold (Biosera), IL-6 (10 ng/ml), and IL-11 (5 ng/ml) with EGM (Lonza Group AG) medium at a volume ratio of 1:1, and adding thereto VEGF (5 ng/mL) and Y-27632 (10 µM).
2) A liver sinusoidal endothelial cell induction medium obtained by mixing HCM (Lonza Group AG) medium supplemented with 5% FBS Gold (Biosera) and OSM (20 ng/ml) with EGM (Lonza Group AG) medium at a volume ratio of 1:1, and adding thereto VEGF (5 ng/mL) and Y-27632 (10 µM).
3) A liver sinusoidal endothelial cell induction medium obtained by mixing HCM (Lonza Group AG) medium supplemented with 5% FBS Gold (Biosera), IL-6 (10 ng/ml), IL-11 (5 ng/ml), and OSM (20 ng/ml) with EGM (Lonza Group AG) medium at a volume ratio of 1:1, and adding thereto VEGF (5 ng/mL) and Y-27632 (10 µM).

The results are shown in Figure 3. As is evident, in the case of using IL-6 and IL-11 as IL-6 family substances, a cell population containing CD32+ICAM1 (CD54)++ LSECs with a given degree of a purity (50% or higher) was obtained. As is also evident, in the case of using three IL-6 family substances, IL-6, IL-11, and OSM, in combination, a cell population containing CD32+ICAM1 (CD54)++ LSECs with a purity (97% or higher) equivalent to or higher than that in the case of using OSM alone was obtained.

## Claims

1. A method for producing sinusoidal endothelial cells from sinusoidal endothelial progenitor cells, the method comprising the step of culturing the sinusoidal endothelial progenitor cells in a medium containing one or more substances selected from the interleukin 6 (IL-6) family.

2. The method according to claim 1, wherein the one or more substances selected from the IL-6 family comprise at least interleukin 6 (IL-6), oncostatin M (OSM), interleukin 11 (IL-11), ciliary neurotrophic factor (CNTF), leukemia inhibitory factor (LIF), cardiotrophin 1 (CT-1), cardiotrophin-like cytokine 1 (CLC), interleukin 27 (IL-27), interleukin 35 (IL-35), interleukin 39 (IL-39), or a combination thereof.

3. The method according to claim 1, wherein the one or more substances selected from the IL-6 family comprise at least IL-6, OSM, IL-11, or a combination thereof.

4. The method according to claim 1, wherein the one or more substances selected from the IL-6 family comprise at least OSM.

5. The method according to claim 1, wherein the sinusoidal endothelial progenitor cells are liver sinusoidal endothelial progenitor cells, and the sinusoidal endothelial cells are liver sinusoidal endothelial cells.

6. The method according to claim 5, wherein the liver sinusoidal endothelial progenitor cells are vitelline venous hemogenic endothelial cells.

7. The method according to claim 1, wherein the medium further contains vascular endothelial growth factor (VEGF).

8. The method according to claim 1, wherein the sinusoidal endothelial progenitor cells are induced from pluripotent stem cells.

9. A cell population comprising sinusoidal endothelial cells obtained by the method according to claim 1.
